(19) Europäisches Patentamt
European Patent Office
Office européen des brevets

(11) Publication number: 0 439 243 A1

# (12) EUROPEAN PATENT APPLICATION

(21) Application number: 91250016.2

(22) Date of filing: 23.01.91

(51) Int. Cl.5: C07D 239/60, C07D 239/34, C07D 239/553, C07D 239/46, C07D 251/30, C07D 251/38, A01N 43/54, A01N 43/66

(30) Priority: 25.01.90 DE 4002365

(43) Date of publication of application: 31.07.91 Bulletin 91/31

(84) Designated Contracting States: AT BE CH DE DK ES FR GB GR IT LI LU NL SE

(71) Applicant: SCHERING AKTIENGESELLSCHAFT
Müllerstrasse 170/178 Postfach 65 03 11
W-1000 Berlin 65(DE)

(72) Inventor: Wegner, Peter, Dr.
Münchener Strasse 3
W-1000 Berlin 28(DE)
Inventor: Krüger, Gabriela, Dr.
Bachstrasse 3
W-1000 Berlin 21(DE)
Inventor: Tarara, Gerhard, Dr.
Sprengelstrasse 36
W-1000 Berlin 65(DE)
Inventor: Harde, Christoph, Dr.
Südwestkorso 61
W-1000 Berlin 41(DE)
Inventor: Gerbling, Klaus Peter, Dr.
Zimmermannstrasse 8
W-1000 Berlin 41(DE)
Inventor: Johann, Gerhard, Dr.
Hermsdorfer Damm 147
W-1000 Berlin 41(DE)
Inventor: Rees, Richard, Dr.
Speerweg 8
W-1000 Berlin 28(DE)

(54) **Substituted beta-pyrimidinyloxy-(thio)- and beta-triazinyloxy(thio)-cycloalkanecarboxylic acid derivatives, processes for their preparation, and their use as herbicides, fungicides and plant growth regulators.**

(57) The invention relates to new substituted α-pyrimidinyloxy(thio)-andα-triazinyloxy(thio)carboxylic acid derivatives of general formula I

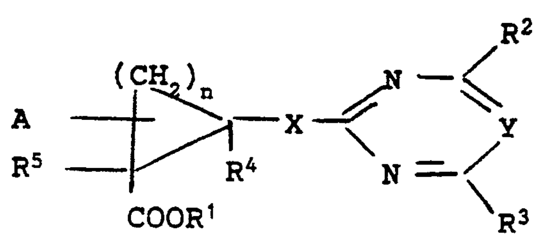

(I)

in which A, $R^{1-5}$, X and Y have the meanings given in the description, processes for their preparation and their use as herbicides, fungicides and plant growth regulants

EP 0 439 243 A1

# SUBSTITUTED β-PYRIMIDINYLOXY(THIO)- AND β-TRIAZINYLOXY(THIO)-CYCLOALKANECARBOXYLIC ACID DERIVATIVES, PROCESS FOR THEIR PREPARATION AND THEIR USE AS HERBICIDES, FUNGICIDES AND PLANT GROWTH REGULATORS

This invention relates to new substituted β-pyrimidinyloxy(thio)-and β-triazinyloxy(thio)-cycloalkanecarboxylic acid derivatives, processes for their preparation and their use as herbicides, fungicides and plant growth regulators.

It is known that pyrimidine derivatives possess herbicidal activity (EP 223 406, 249 707, 249 708, 287 072 and 287 079). However, the herbicidal activity of these known compounds is often insufficient or selectivity problems are seen in important crops.

It has now been found that substituted β-pyrimidinyloxy(thio)-and β-triazinyloxy(thio)-cycloalkanecarboxylic acid derivatives of general formula I

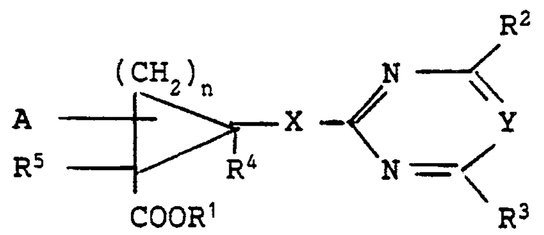

(I)

in which

$R^1$ is

hydrogen, $C_1$-$C_{12}$-alkyl (optionally substituted by hydroxy, halogen, $C_1$-$C_4$-alkoxy, $C_1$-$C_4$-alkylthio, $C_3$-$C_6$-cycloalkyl, benzyl, furyl, tetrahydrofuryl, dioxanyl, dioxolanyl, phenyl, halophenyl, $C_1$-$C_4$-alkylphenyl, $C_1$-$C_4$-alkoxyphenyl, nitrophenyl, cyano, carboxy, $C_1$-$C_4$-alkoxycarbonyl, $C_1$-$C_4$-alkylamino or di-$C_1$-$C_4$-alkylamino), $C_2$-$C_{12}$-alkyl, interrupted by one or more oxygen or sulphur atoms and optionally substituted by hydroxy, halogen, $C_1$-$C_4$-alkoxy, $C_1$-$C_4$-alkylthio, $C_3$-$C_6$-cycloalkyl, benzyl, furyl, tetrahydrofuryl, dioxanyl, dioxolanyl, phenyl, halophenyl, $C_1$-$C_4$-alkylphenyl, $C_1$-$C_4$-alkoxyphenyl, nitrophenyl, cyano, carboxy, $C_1$-$C_4$-alkoxycarbonyl, $C_1$-$C_4$-alkylamino or di-$C_1$-$C_4$-alkylamino, a $C_3$-$C_8$-alkenyl or $C_3$-$C_8$-alkynyl group (each of which is optionally substituted by $C_1$-$C_3$-alkoxy, phenyl or halogen), $C_3$-$C_6$-cycloalkyl, optionally substituted by methyl and/or optionally interrupted by one or two oxygen atoms, succinimido, phthalimido, di-$C_1$-$C_4$-alkylmethylenamino, $C_1$-$C_4$-alkylphenylmethylenamino or $C_3$-$C_6$-cycloalkylidenamino;

$R^2$ and $R^3$,

which may be the same or different, are $C_1$-$C_4$-alkyl, halo-$C_1$-$C_4$-alkyl, $C_1$-$C_4$-alkoxy, halo-$C_1$-$C_4$-alkoxy, $C_1$-$C_4$-alkoxy-$C_1$-$C_4$-alkyl, $C_1$-$C_4$-alkylthio, $C_1$-$C_4$-alkylamino, di-$C_1$-$C_4$-alkylamino or halogen;

$R^4$ and $R^5$,

which may be the same or different, are hydrogen, halogen, $C_1$-$C_{10}$-alkyl, $C_2$-$C_{10}$-alkenyl or $C_2$-$C_{10}$-alkynyl group (each of which is optionally substituted by one or more of the same or different $C_1$-$C_4$-alkoxy, $C_1$-$C_4$-alkylthio, amino, $C_1$-$C_4$-alkylamino, di-$C_1$-$C_4$-alkylamino, nitro, halogen or phenyl), or a $C_3$-$C_8$-cycloalkyl or $C_4$-$C_8$-cycloalkenyl group (each of which is optionally substituted by one or more of the same or different $C_1$-$C_4$-alkyl, $C_1$-$C_4$-alkoxy, $C_1$-$C_4$-alkylthio, amino, $C_1$-$C_4$-alkylamino, di-$C_1$-$C_4$-alkylamino, nitro, trifluoromethyl, halogen or phenyl) or is phenyl;

A is

hydrogen, $C_1$-$C_{10}$-alkyl, $C_2$-$C_{10}$-alkenyl or $C_2$-$C_{10}$-alkynyl group (each of which is optionally substituted by one or more of the same or different $C_1$-$C_4$-alkoxy, $C_1$-$C_4$-alkylthio, amino, $C_1$-$C_4$-alkylamino, di-$C_1$-$C_4$-alkylamino, nitro, halogen or phenyl), or a $C_3$-$C_8$-cycloalkyl or $C_4$-$C_8$-cycloalkenyl group (each of which is optionally substituted by one or more of the same or different $C_1$-$C_4$-alkyl, $C_1$-$C_4$-alkoxy, $C_1$-$C_4$-alkylthio, amino, $C_1$-$C_4$-alkylamino, di-$C_1$-$C_4$-alkylamino, nitro, trifluoromethyl, halogen or phenyl) or is phenyl;

X is oxygen or sulphur;

Y is methine or nitrogen; and

n is 3, 4, 5 or 6;

as well as their alkali metal, alkaline earth metal and organic ammonium salts, and their optical isomers, show interesting herbicidal, fungicidal and plant growth regulant activity.

A preferred group of compounds are those where $R^1$ hydrogen, methyl, ethyl, phthalimido, succinimido, cyclohexylidenamino or the groups

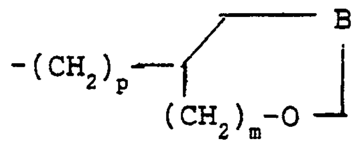 or 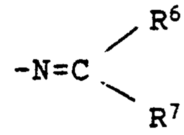

$R^2$ is methoxy, methyl, chloro or dimethylamino,
$R^3$ is methoxy or methyl,
$R^4$ is hydrogen or methyl,
$R^5$ is hydrogen, methyl or chloro,
$R^6$ is methyl,
A is hydrogen or $C_{1-4}$-alkyl,
B is oxygen or methylene,
X is oxygen,
Y is methine,
n is 3, 4 or 5,
m is 0 or 1, and
p is 0 or 1.

A particularly preferred group of compounds are those where
$R^1$ is hydrogen, methyl or ethyl,
$R^2$ and $R^3$ are methoxy,
$R^4$ and $R^5$ are hydrogen,
A is hydrogen,
X is oxygen,
Y is methine,
and n is 4.

The compounds of formula I can exist in various enantiomeric, diastereoisomeric or geometric forms and these are all within the scope of the invention.

The expression "halogen" means fluorine, chlorine, bromine and iodine. By the term alkali metal is meant lithium, sodium or potassium and by the term alkaline earth metal is meant calcium, strontium or barium.

The compounds of the invention of general formula I can be prepared for example

A) by reacting a compound of general formula II

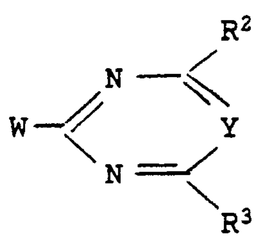

(II)

in which $R^2$, $R^3$ and Y have the meanings given under general formula I and W is halogen, alkylsulphonyl or phenylsulphonyl, with a compound of general formula III

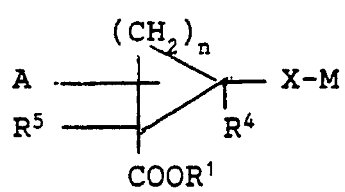

(III)

in which A, $R^1$, $R^4$, $R^5$, X and n have the meanings given under general formula I, in a suitable solvent in the presence of a suitable base, or

B) by reacting a compound of general formula VIII

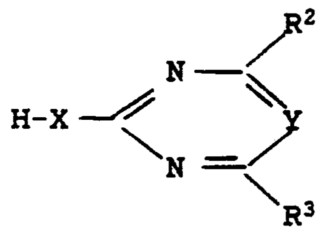

(VIII)

in which $R^2$, $R^3$, X and Y have the meanings given under general formula I, with a compound of general formula IX

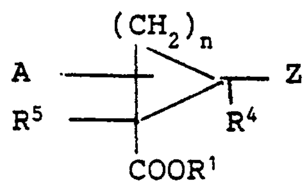

(IX)

in which A, $R^1$, $R^4$, $R^5$, X and n have the meanings given under general formula I and Z is halogen or alkylsulphonyloxy, in a suitable solvent in the presence of a suitable base,

and if desired (i) a compound of general formula I in which $R^1$ is hydrogen, $C_1$-$C_4$-alkyl or benzyl, so obtained, is reacted with an alkali metal base or an alkaline earth metal base, in a suitable polar solvent, to give a compound of general formula I in which $R^1$ is an alkali metal atom or one equivalent of an alkaline earth metal atom, (ii) and/or if desired, a compound of formula I in which $R^1$ is an alkali metal atom or one equivalent of an alkaline earth metal atom, is reacted with a suitable acid in a suitable solvent to give a compound of general formula I in which $R^1$ is hydrogen, (iii) and/or if desired, a compound of formula I in which $R^1$ is hydrogen is reacted with a suitable base in a suitable solvent to give a compound of general formula I in which $R^1$ is an alkali metal atom or one equivalent of an alkaline earth metal atom or an organic ammonium group.

The individual process variants are preferably carried out in the presence of a diluent. For this, a solvent which is inert to the reactants is used.

Suitable solvents include water, aliphatic, alicyclic and aromatic hydrocarbons, that can be optionally chlorinated, such as for example hexane, cyclohexane, petroleum ether, ligroin, benzene, toluene, xylene, methylene chloride, chloroform, carbon tetrachloride, ethylene chloride and trichloroethane, ethers, such as for example diisopropyl ether, dibutyl ether, propylene oxide, dioxane and tetrahydrofuran; ketones, such as for example acetone, methyl ethyl ketone, methyl isopropyl ketone, nitriles, such as for example acetonitrile and propionitrile, alcohols, such as for example methanol, ethanol, isopropanol, butanol and ethylene glycol, esters, such as for example ethyl acetate and amyl acetate, amides, such as for example dimethylformamide and dimethylacetamide, sulphones and sulphoxides, such as for example dimethyl sulphoxide, and bases, such as for example pyridine.

The presence of a catalyst can be an advantage. Suitable catalysts include potassium iodide and onium compounds. such as quaternary ammonium, phosphonium and arsonium compounds as well as sulphonium compounds. Also suitable are polyglycol ethers, especially cyclic ethers, such as 18-crown-6, and tertiary amines, such as for example tributylamine. Preferred compounds are quaternary ammonium compounds, such as for example benzyltriethylammonium chloride and tetrabutylammonium bromide.

The reactions can be carried out under atmospheric pressure but if desired higher or lower pressures can be used.

The process variant A) is preferably carried out in an aromatic hydrocarbon, such as benzene, toluene or xylene, a halogenated hydrocarbon, such as methylene chloride or chloroform, an alcohol, such as methanol, ethanol or isopropanol, an ether, such as for example diethyl ether, diisopropyl ether, tetrahydrofuran or dioxane, a ketone, such as for example acetone or methyl ethyl ketone, an ester, such as methyl acetate or ethyl acetate, or a polar aprotic solvent, such as dimethylformamide, dimethylacetamide or dimethyl sulphoxide, or another solvent, such as acetonitrile or water.

Bases that can be used include an alkali metal, such as sodium or potassium, an alkali metal or alkaline earth metal hydride, such as sodium hydride, potassium hydride or calcium hydride, a carbonate, such as sodium carbonate or potassium carbonate, or a metal hydroxide, such as sodium hydroxide or potassium hydroxide.

The reaction is suitably carried out between room temperature and the boiling point of the particular solvent or solvent mixture. The reaction time lies between 1 and 24 hours.

The reaction can also be carried out in the absence of a solvent, at a temperature between 120 and 160 ° C, by using as alkali metal carbonate, such as anhydrous potassium carbonate.

The process variants (i) and (ii) are preferably carried out in an alcohol, such as ethanol, propanol or isopropanol, a ketone, such as acetone or methyl ethyl ketone, water or a mixture of water and a polar solvent.

Bases that can be used include carbonates, such as sodium carbonate, potassium carbonate or calcium carbonate, and metal hydroxides, such as sodium hydroxide or potassium hydroxide.

The temperature of the reaction falls within room temperature and the boiling point of the particular solvent or solvent mixture. The reaction time lies between 0.5 and 36 hours.

When converting an ester in which $R^1$ is benzyl to the free acid a catalytic reduction (hydrogenation) can also be used.

Suitable solvents for process variant (iii) include hydrocarbons, such as benzene, toluene or xylene, halogenated hydrocarbons, such as methylene chloride or chloroform, alcohols, such as methanol, ethanol or isopropanol, ethers, such as for example diethyl ether, diisopropyl ether, tetrahydrofuran or 1,4-dioxane, ketones, such as acetone or methyl ethyl ketone, esters, such as methyl acetate or ethyl acetate, or nitriles, such as acetonitrile.

Bases that can be used include an alkali metal, such as sodium or potassium, an alkali metal or alkaline earth metal hydride, such as sodium hydride, potassium hydride or calcium hydride, a carbonate, such as sodium carbonate, potassium carbonate or calcium carbonate, or a metal hydroxide, such as sodium hydroxide or potassium hydroxide.

Organic ammonium bases that can be used include for example, ammonia an alkylamine (primary amine), a dialkylamine (secondary amine) or a trialkylamine (tertiary amine).

The temperature of the reaction falls within room temperature and the boiling point of the particular solvent or solvent mixture. The reaction time lies between 5 minutes and 10 hours.

Suitable solvents for process variant B) include hydrocarbons, such as benzene, toluene or xylene, halogenated hydrocarbons, such as methylene chloride or chloroform, ethers, such as for example diethyl ether, diisopropyl ether, tetrahydrofuran or 1,4-dioxane, ketones, such as acetone or methyl ethyl ketone, esters, such as methyl acetate or ethyl acetate, or polar aprotic solvents, such as dimethyl-formamide, dimethylacetamide or dimethyl sulphoxide, and other solvents, such as acetonitrile or water.

Bases that can be used include an alkali metal, such as sodium or potassium, an alkali metal or alkaline earth metal hydride, such as sodium hydride, potassium hydride or calcium hydride, a carbonate, such as sodium carbonate, potassium carbonate, sodium hydrogen carbonate or potassium hydrogen carbonate, or a metal hydroxide, such as sodium hydroxide or potassium hydroxide.

The temperature of the reaction falls within room temperature and the boiling point of the particular solvent or solvent mixture. The reaction time lies between 5 minutes and 24 hours. Compounds of general formula III are described in the literature or can be prepared by methods analogous to those described in the literature.

The compounds of the invention prepared by these processes can be isolated from the reaction mixtures in conventional manner, for example by distillation of the solvent at normal or reduced pressure, by precipitation with water or by extraction.

A higher level of purity can be achieved as a rule by column chromatography as well as by fractional distillation or crystallisation.

The compounds of the invention are, as a rule, colourless and odourless liquids or crystals that are soluble in water, slightly soluble in aliphatic hydrocarbons such as petroleum ether, hexane, pentane and cyclohexane and highly soluble in halogenated hydrocarbons, such as chloroform, methylene chloride and carbon tetrachloride, aromatic hydrocarbons, such as benzene, toluene and xylene, ethers, such as diethyl ether, tetrahydrofuran and dioxane, nitriles, such as acetonitrile, alcohols, such as methanol and ethanol, amides, such as dimethylformamide, and sulphoxides, such as dimethyl sulphoxide.

The compounds of the invention show a good herbicidal activity in broad leaved weeds and grasses. A selective use in various crops is possible, for example in such as rape, beets, soya beans, cotton, rice, barley, wheat and other cereals. Individual active substances are particularly suitable as selective herbicides in beet, cotton, soya and cereals. However the compounds can be used for control of weeds in permanent crops, such as for example forestry, ornamental trees, fruit, vine, citrus, nut, banana, coffee, tea, rubber, oil palm, cocoa, berry fruit and hop plantations and for the selective control of weeds in annual crops.

The compounds of the invention can used for example against the following plant species:

Dicotyledonous weeds of the species Sinapis, Lepidium, Galium, Stellaria, Matricaria, Anthemis, Galinsoga,

Chenopodium, Brassica, Urtica, Seneoio, Amaranthus, Portulaca, Xanthium, Convolvulus, Ipomoea, Polygonum, Sesbania, Ambrosia, Cirsium, Carduus, Sonchus, Solanum, Rorippa, Lamium, Veronica, Abutilon, Datura, Viola, Galeopsis, Papaver, Centaurea and Chrysanthemum.

Monocotyledonous weeds of the species Avena, Alopecurus, Echinochloa, Setaria, Panicum, Digitaria, Poa, Eleusine, Brachiaria, Lolium, Bromus, Cyperus, Agropyron, Sagittaria, Monocharia, Fimbristylis, Eleocharis, Ischaemum and Apera.

The rates of use vary depending on the manner of pre- and postemergent use between 0.001 and 5 kg/ha.

The compounds of the invention can also be used as defoliants, desiccants and as total herbicides. They also influence plant growth and can thus be used to influence plant growth of crops. Some compounds also show fungicidal activity.

The compounds of the invention can be used either alone or in admixture with one another or with other active agents. Optionally, other plant-protective agents or pesticides can be added, depending on the purpose for the treatment. When it is desired to broaden the spectrum of activity, other herbicides can also be added. Herbicidally active mixing partners suitable in this connection include for example, the active agents listed in Weed Abstracts, vol. 38, No.3 (1989) under the heading "Lists of common names and abbreviations employed for currently used herbicides and plant growth regulators in Weed Abstracts".

An improvement in the intensity and speed of action can be obtained, for example, by addition of suitable adjuvants, such as organic solvents, wetting agents and oils. Such additives may allow a decrease in the dose.

The designated active ingredients or their mixtures can suitably be used, for example, as powders, dusts, granules, solutions, emulsions or suspensions, with the addition of liquid and/or solid carriers and/or diluents and, optionally, binding, wetting, emulsifying and/or dispersing adjuvants.

Suitable liquid carriers are, for example aliphatic and aromatic hydrocarbons, such as benzene, toluene, xylene, cyclohexanone, isophorone, dimethyl sulphoxide, dimethylformamide and other mineral-oil fractions and plant oils.

Suitable solid carriers include mineral earths, e.g. bentonite, silica gel, talc, kaolin, attapulgite, limestone, silicic acid and plant products, e.g. flours.

As surface-active agents there can be used for example calcium lignosulphonate, polyoxyethylenealkylphenyl ethers, naphthalenesulphonic acids and their salts, phenolsulphonic acids and their salts, formaldehyde condensates, fatty alcohol sulphates, as well as substituted benzenesulphonic acids and their salts.

The percentage of the active ingredient(s) in the various preparations can vary within wide limits. For example, the compositions can contain about 10 to 90 percent by weight active ingredients, and about 90 to 10 percent by weight liquid or solid carriers, as well as, optionally up to 20 percent by weight of surfactant.

The agents can be applied in customary fashion, for example with water as the carrier in spray mixture volumes of approximately 100 to 1,000 l/ha. The agents can be applied using low-volume or ultra-low-volume techniques or in the form of so-called microgranules.

The preparation of these formulations can be carried out in known manner, for example by milling or mixing processes. Optionally, individual components can be mixed just before use for example by the so-called commonly used tank-mixing method.

Formulations can be prepared, for example, from the following ingredients.

A) Wettable Powder

1)

25 percent by weight active ingredient
60 percent by weight kaolin
10 percent by weight silicic acid
5 percent by weight of a mixture of calcium lignosulphonate and the sodium salt of N-methyl-N-oleyltaurine

2)

40 percent by weight active ingredient
25 percent by weight bentonite
25 percent by weight colloidal silicic acid
10 percent by weight of a mixture of calcium lignosulphonate and alkylphenyl polyglycol ether

B) Paste

45 percent by weight active ingredient
5 percent by weight sodium aluminium silicate
15 percent by weight cetyl polyglycol ether with 8 moles of ethylene oxide

2 percent by weight spindle oil
10 percent by weight polyethylene glycol
23 percent by weight water

C) Emulsifiable Concentrate
25 percent by weight active ingredient
15 percent by weight cyclohexanone
55 percent by weight xylene
5 percent by weight of a mixture of calcium dodecylbenzenesulphonate and nonylphenolpolyoxyethylene.

The following Examples illustrate the preparation of compounds of the invention.

Example 1

Methyl cis,trans-2-(4,6-dimethoxypyrimidin-2-yloxy)-cyclohexanecarboxylate

2.3 g (14.7 mmol) Methyl 2-hydroxycyclohexanecarboxylate was treated with 2.2 g (16 mmol) potassium carbonate in 20 ml dimethylformamide. 3 g (14.7 mmol) 4,6-Dimethoxy-2-methylsulphonylpyrimidine was added to the solution and the mixture stirred for 10 hours at 50°C. The reaction mixture was concentrated, poured into 200 ml ice-water and extracted with ethyl acetate. The ethyl acetate phase was dried over magnesium sulphate and concentrated. The residue was purified by silica-gel column chromatography using hexane/ethyl acetate (95:5) as eluent to give a colourless oil.
Yield: 1.68 g = 39% of theory
$n_D^{20}$ = 1.490
This was present as a cis/trans isomer mixture (60:40, nmr).

Example 2

Cis,trans-2-(4,6-dimethoxypyrimidin-2-yloxy)cyclohexanecarboxylic acid

2.0 g (14 mmol) 2-Hydroxycyclohexanecarboxylic acid was treated with 3.15 g (28 mmol) potassium t-butylate in 20 ml dimethylformamide. 3.15 g (15 mmol) 4,6-Dimethoxy-2-methylsulphonylpyrimidine was added to the solution and the mixture stirred for 24 hours at room temperature. The reaction mixture was concentrated, poured into 200 ml ice-water and extracted with ethyl acetate. The ethyl acetate phase was dried over magnesium sulphate and concentrated. The residue was purified by silica-gel column chromatography using hexane/ethyl acetate (95:5 to 1:1) as eluent.
Yield: 0.53 g = 13% of theory
mp : 133.3°C
This was present as a cis/trans isomer mixture (60:40, nmr).

Example 3

(±)-trans-2-(4,6-dimethoxypyrimidin-2-yloxy)cyclohexanecarboxylic acid

1.3 g (4.5 mmol) Methyl (±)-trans-2-(4,6-dimethoxypyrimidin-2-yloxy)cyclohexanecarboxylate was dissolved in 20 ml water/methanol (1:1), treated with 4.4 g potassium hydroxide at room temperature and the mixture stirred at 50°C for 3 hours. The mixture was concentrated, the residue taken up in 10 ml water and extracted with 50 ml ethyl acetate. The aqueous phase was acidified with 2N hydrochloric acid until it was pH 5 and extracted three times with ethyl acetate. After drying over magnesium sulphate, the ethyl acetate phase was concentrated and the residue purified by silica-gel column chromatography using hexane/acetone (8:2) as eluent.
Yield: 510 mg = 41% of theory
mp: 131°C
In a similar manner, the following compounds of the invention were prepared.

| Ex | Compound | Physical Constant $n_D^{20}$ | mp(°C) |
|---|---|---|---|
| 4 | Methyl (±)-cis-2-(4,6-dimethoxypyrimidin-2-yloxy)cycloheptanecarboxylate | 1.4968 | |
| 5 | Methyl (±)-trans-2-(4,6-dimethoxypyrimidin-2-yloxy)cycloheptanecarboxylate | 1.502 | |
| 6 | Methyl (±)-cis-2-(4,6-dimethoxypyrimidin-2-yloxy)cyclohexanecarboxylate | 1.500 | |
| 7 | Methyl (±)-trans-2-(4,6-dimethoxypyrimidin-2-yloxy)cyclohexanecarboxylate | 1.481 | |
| 8 | (±)-cis-2-(4,6-Dimethoxypyrimidin-2-yloxy)cycloheptanecarboxylic acid | | 85.5 |
| 9 | (±)-cis-2-(4,6-Dimethoxypyrimidin-2-yloxy)cyclohexanecarboxylic acid | | 130.2 |
| 10 | Methyl cis,trans-2-(4,6-dimethoxypyrimidin-2-yloxy)cyclopentanecarboxylate (cis/trans isomer mixture 50:50, nmr) | 1.5060 | |
| 11 | Methyl (±)-trans-2-(4,6-dimethoxypyrimidin-2-yloxy)cyclopentanecarboxylate | 1.5067 | |
| 12 | Methyl (±)-cis-2-(4,6-dimethoxypyrimidin-2-yloxy)cyclopentanecarboxylate | 1.5062 | |
| 13 | Methyl (±)-cis,trans-2-(4,6-dimethyl pyrimidin-2-yloxy)cyclohexanecarboxylate (cis/trans isomer mixture 60:40, nmr) | 1.5096 | |

| Ex | Compound | Physical Constant $n_D^{20}$ | mp(°C) |
|---|---|---|---|
| 14 | Ethyl (±)-cis,trans-2-(4-chloro-6-methoxy-pyrimidin-2-yloxy)cyclohexanecarboxylate | 1.5108 | |
| 15 | (±)-1,2-trans-2-(4,6-dimethoxypyrimidin-2-yloxy)-5-t-butylcyclohexanecarboxylic acid | | 225 |
| 16 | (±)-1,2-cis-2-(4,6-dimethoxypyrimidin-2-yloxy)-5-t-butylcyclohexanecarboxylic acid | | 189-190 |
| 17 | Ethyl (±)-1,2-cis-2-(4,6-dimethoxy-pyrimidin-2-yloxy)-3-methylcyclohexane-carboxylate | 1.4971 | |
| 18 | Ethyl (±)-1,2-cis,trans-2-(4,6-dimethoxy-pyrimidin-2-yloxy)-3-methylcyclohexane-carboxylate (cis/trans isomer mixture 75:25, nmr) | 1.4960 | |
| 19 | (±)-cis-2-(4-dimethylamino-6-methoxy-pyrimidin-2-yloxy)cyclohexanecarboxylic acid | | 150 |
| 20 | 2-Tetrahydrofurfuryl (±)-trans-2-(4,6-dimethoxy-pyrimidin-2-yloxy)cyclohexanecarboxylate | | * |
| 21 | (±)-trans-2-(4,6-dimethoxypyrimidin-2-yloxy)-cyclohexanecarboxylic acid acetoneoxime ester | | ** |
| 22 | Ethyl (±)-trans-2-(4,6-dimethoxypyrimidin-2-yloxy)cyclohexanecarboxylate | 1.5021 | |

| Ex | Compound | Physical Constant $n_D^{20}$ | mp(°C) |
|---|---|---|---|
| 23 | Ethyl (±)-1-chloro-2-(4,6-dimethoxypyrimidin-2-yloxy)cyclohexanecarboxylate | | 67-70 |
| 24 | Succinimido (±)-trans-2-(4,6-dimethoxypyrimidin-2-yloxy)cyclohexanecarboxylate | | 165-6 |
| 25 | Phthalimido (±)-trans-2-(4,6-dimethoxypyrimidin-2-yloxy)cyclohexanecarboxylate | | 145-6 |
| 26 | (1,3-Dioxolan-4-yl)methyl (±)-trans-2-(4,6-dimethoxypyrimidin-2-yloxy)-cyclohexanecarboxylate | 1.5028 | |
| 27 | 1,3-Dioxan-5-yl (±)-trans-2-(4,6-dimethoxypyrimidin-2-yloxy)cyclohexanecarboxylate | 1.5080 | |
| 28 | (±)-1-Chloro-2-(4,6-dimethoxypyrimidin-2-yloxy)cyclohexanecarboxylic acid | | 167-174 |
| 29 | 1,3-Difluoroprop-2-yl (±)-trans-2-(4,6-dimethoxypyrimidin-2-yloxy)-cyclohexanecarboxylate | 1.4827 | |
| 30 | Ethyl (±)-2-(4,6-dimethoxypyrimidin-2-yloxy)-1-methylcyclohexanecarboxylate (1. diastereoisomer) | 1.4980 | |
| 31 | Ethyl (±)-2-(4,6-dimethoxypyrimidin-2-yloxy)-1-methylcyclohexanecarboxylate (2. diastereoisomer) | 1.4967 | |

| Ex | Compound | Physical Constant $n_D^{20}$ | mp(°C) |
|---|---|---|---|
| 32 | Ethyl (±)-2-(4,6-dimethoxypyrimidin-2-yloxy)-1,2-dimethylcyclohexanecarboxylate | 1.5035 | |
| 33 | (±)-trans-2-(4,6-dimethoxypyrimidin-2-yloxy)cyclohexanecarboxylic acid cyclohexaneoxime ester | 1.5188 | |
| 34 | (±)-trans-2-(4,6-dimethoxypyrimidin-2-yloxy)cyclohexanecarboxylic acid acetophenoneoxime ester | | 119-120 |

* nmr: (d-DMSO): δ 1.3-1.6(m,5H), 1.6-1.9(m,5H), 1.95(m,1H), 2.2 (m,1H), 2.65 (ddd,1H), 3.55-3.75 (m,2H), 3.85(s,6H), 3.8-4.05(m,3H), 5.12 (ddd,1H), 5.85(s,1H)

** nmr: (d-DMSO): δ 1.25-1.45(m,3H), 1.55(m,1H), 1.65-1.8(m,2H), 1.9(s,3H), 1.95(s,3H), 2.05(m,1H), 2.25(m,1H), 2.75(ddd,1H), 3.85(s,6H), 5.12 (ddd,1H), 5.82(s,1H)

The following examples illustrate the possibilities for use of the compounds of the invention.

In these Examples the abbreviations have the following meanings:

ABUTH = Abutilon theophrasti
ALOMY = Alopecurus myosuroides
BROTE = Bromus tectorum
CYPES = Cyperus esculentus
GALAP = Galium aparine
MATCH = Matricaria chamomilla
PANSS = Panicum maximum
POLSS = Polygonum sp.
SEBEX = Sesbania exaltata
SETVI = Setaria viridis
SORHA = Sorghum halepense
SOLSS = Solanum sp.
VERPE = Veronica persica
VIOSS = Viola spp

In these Examples damage to plants is assessed on a score of 0 to 4, in which the scores have the following meanings:

0 = no damage

1 = 1 - 24% damage
2 = 25 - 74% damage
3 = 75 - 89% damage
4 = 90 - 100% damage

Example A

In a greenhouse, the noted plant species were treated pre-emergently with the noted compounds, at a rate of 0.1 kg active ingredient/ha. The compounds were sprayed evenly over the soil as emulsions or suspensions in 500 litres water/ha. Three weeks after the treatment, the compounds of the invention showed good activity against the weeds. The comparison material did not show the same high activity.

| Compound of the invention | GALAP | POLSS |
|---|---|---|
| Example 1 | 3 | 3 |
| Example 2 | 3 | 3 |
| Example 11 | 3 | - |
| Example 20 | - | 3 |
| Example 21 | 4 | 3 |
| Example 22 | 3 | 3 |
| Untreated | 0 | 0 |
| Comparison (EP 223 406) Example 4 | 2 | 2 |

Example B

In a greenhouse, the noted plant species were treated post-emergently with the noted compounds of the invention, at a rate of 0.3 kg active ingredient/ha. The compounds of the invention were sprayed evenly over the plants as emulsions in 500 litres water/ha. Two weeks after the treatment, the compounds of the invention showed excellent activity against the weeds. The comparison material did not show similar high efficacy.

| Compound of the invention | ALOMY | BROTE | SETVI | PANSS | SORHA | ABUTH | GALAP | MATCH | POLSS | SEBEX | SOLSS | VERPE | VIOSS |
|---|---|---|---|---|---|---|---|---|---|---|---|---|---|
| Example 1 | 3 | 2 | 3 | 3 | 3 | 4 | 2 | 2 | 3 | 4 | 2 | 3 | 3 |
| Example 2 | 3 | 3 | 3 | 3 | 3 | 4 | 3 | 3 | 3 | 4 | 3 | 3 | 3 |
| Example 7 | 3 | 2 | 3 | 3 | 3 | 4 | 3 | 3 | 3 | 3 | 2 | 3 | 3 |
| Example 11 | 3 | - | - | - | - | 3 | 3 | - | - | 4 | 3 | - | - |
| Example 14 | 3 | 3 | - | - | 3 | 3 | 3 | - | 3 | 3 | 3 | 3 | 3 |
| Example 20 | 3 | 3 | 3 | 3 | 3 | 3 | 4 | 3 | 3 | 3 | 3 | 3 | 3 |
| Example 21 | 3 | 3 | 3 | 3 | 3 | 4 | 4 | 4 | 3 | 3 | 4 | 4 | 4 |
| Example 22 | 3 | 3 | 3 | 3 | 3 | 3 | 3 | 3 | 3 | 3 | 3 | 4 | 3 |
| Untreated | 0 | 0 | 0 | 0 | 0 | 0 | 0 | 0 | 0 | 0 | 0 | 0 | 0 |

Example C

In a greenhouse, the noted plant species were treated pre-emergently with the noted compounds, at a rate of 0.1 kg active ingredient/ha. The compounds were sprayed evenly over the soil as emulsions or suspensions in 500 litres water/ha. Three weeks after the treatment, the compounds of the invention showed excellent activity against the weeds. The comparison material did not show the same high activity.

| Compound of the invention | CYPES | MATCH |
|---|---|---|
| Example 7 | 3 | 3 |
| Example 14 | - | 3 |
| Example 20 | 3 | 3 |
| Example 21 | - | 3 |
| Example 22 | - | 3 |
| Untreated | 0 | 0 |
| Comparison (EP 223 406) Example 4 | 2 | 0 |

## Claims

1. Substituted $\beta$-pyrimidinyloxy-(thio)- and $\beta$-triazinyloxy(thio)cycloalkanecarboxylic acid derivatives of general formula I

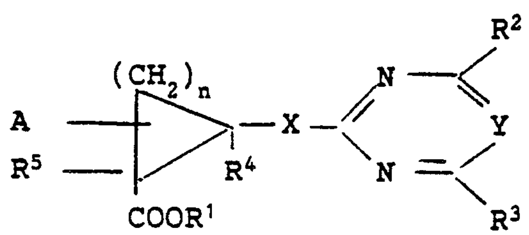

(I)

in which

$R^1$ is

hydrogen, $C_1$-$C_{12}$-alkyl (optionally substituted by hydroxy, halogen, $C_1$-$C_4$-alkoxy, $C_1$-$C_4$-alkylthio, $C_3$-$C_6$-cycloalkyl, benzyl, furyl, tetrahydrofuryl, dioxanyl, dioxolanyl, phenyl, halophenyl, $C_1$-$C_4$-alkylphenyl, $C_1$-$C_4$-alkoxyphenyl, nitrophenyl, cyano, carboxy, $C_1$-$C_4$-alkoxycarbonyl, $C_1$-$C_4$-alkylamino or di-$C_1$-$C_4$-alkylamino), $C_2$-$C_{12}$-alkyl, interrupted by one or more oxygen or sulphur atoms and optionally substituted by hydroxy, halogen, $C_1$-$C_4$-alkoxy, $C_1$-$C_4$-alkylthio, $C_3$-$C_6$-cycloalkyl, benzyl, furyl, tetrahydrofuryl, dioxanyl, dioxolanyl, phenyl, halophenyl, $C_1$-$C_4$-alkylphenyl, $C_1$-$C_4$-alkoxyphenyl, nitrophenyl, cyano, carboxy, $C_1$-$C_4$-alkoxycarbonyl, $C_1$-$C_4$-alkylamino or di-$C_1$-$C_4$-alkylamino, a $C_3$-$C_8$-alkenyl or $C_3$-$C_8$-alkynyl group (each of which is optionally substituted by $C_1$-$C_3$-alkoxy, phenyl or halogen), $C_3$-$C_6$-cycloalkyl, optionally substituted by methyl and/or optionally interrupted by one or two oxygen atoms, succinimido, phthalimido, di-$C_1$-$C_4$-alkylmethylenamino, $C_1$-$C_4$-alkylphenylmethylenamino or $C_3$-$C_6$-cycloalkylidenamino;

$R^2$ and $R^3$,

which may be the same or different, are $C_1$-$C_4$-alkyl, halo-$C_1$-$C_4$-alkyl, $C_1$-$C_4$-alkoxy, halo-$C_1$-$C_4$-alkoxy, $C_1$-$C_4$-alkoxy-$C_1$-$C_4$-alkyl, $C_1$-$C_4$-alkylthio, $C_1$-$C_4$-alkylamino, di-$C_1$-$C_4$-alkylamino or halogen;

$R^4$ and $R^5$,
which may be the same or different, are hydrogen, halogen, $C_1$-$C_{10}$-alkyl, $C_2$-$C_{10}$-alkenyl or $C_2$-$C_{10}$-alkynyl group (each of which is optionally substituted by one or more of the same or different $C_1$-$C_4$-alkoxy, $C_1$-$C_4$-alkylthio, amino, $C_1$-$C_4$-alkylamino, di-$C_1$-$C_4$-alkylamino, nitro, halogen or phenyl), or a $C_3$-$C_8$-cycloalkyl or $C_4$-$C_8$-cycloalkenyl group (each of which is optionally substituted by one or more of the same or different $C_1$-$C_4$-alkyl, $C_1$-$C_4$-alkoxy, $C_1$-$C_4$-alkylthio, amino, $C_1$-$C_4$-alkylamino, di-$C_1$-$C_4$-alkylamino, nitro, trifluoromethyl, halogen or phenyl) or is phenyl;
A is
hydrogen, $C_1$-$C_{10}$-alkyl, $C_2$-$C_{10}$-alkenyl or $C_2$-$C_{10}$-alkynyl group (each of which is optionally substituted by one or more of the same or different $C_1$-$C_4$-alkoxy, $C_1$-$C_4$-alkylthio, amino, $C_1$-$C_4$-alkylamino, di-$C_1$-$C_4$-alkylamino, nitro, halogen or phenyl), or a $C_3$-$C_8$-cycloalkyl or $C_4$-$C_8$-cycloalkenyl group (each of which is optionally substituted by one or more of the same or different $C_1$-$C_4$-alkyl, $C_1$-$C_4$-alkoxy, $C_1$-$C_4$-alkylthio, amino, $C_1$-$C_4$-alkylamino, di-$C_1$-$C_4$-alkylamino, nitro, trifluoromethyl, halogen or phenyl) or is phenyl;
X is oxygen or sulphur;
Y is methine or nitrogen; and
n is 3, 4, 5 or 6;
as well as their alkali metal, alkaline earth metal and organic ammonium salts, and their optical isomers.

**2.** Compounds according to claim 1 in which $R^1$ hydrogen, methyl, ethyl, phthalimido, succinimido, cyclohexylidenamino or the groups

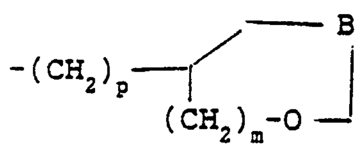

or

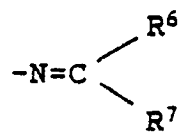

$R^2$ is methoxy, methyl, chloro or dimethylamino,
$R^3$ is methoxy or methyl,
$R^4$ is hydrogen or methyl,
$R^5$ is hydrogen, methyl or chloro,
$R^6$ is methyl,
A is hydrogen or $C_{1-4}$-alkyl,
B is oxygen or methylene,
X is oxygen,
Y is methine,
n is 3, 4 or 5,
m is 0 or 1, and
p is 0 or 1.

**3.** Compounds according to claim 2 in which
$R^1$ is hydrogen, methyl or ethyl,
$R^2$ and $R^3$ are methoxy,
$R^4$ and $R^5$ are hydrogen,
A is hydrogen,
X is oxygen,
Y is methine,
and n is 4.

**4.** A herbicidal composition which comprises a compound according to any one of claims 1 to 3, in admixture with agriculturally acceptable carriers or diluents.

**5.** A method of combating weeds which comprises applying to the weeds or their locus a compound according to any one of claims 1 to 3.

European Patent Office

# EUROPEAN SEARCH REPORT

Application Number

EP 91250016.2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | EP - A2 - 0 315 889 (KUMIAI) * Claims 1,10,11 * -- | 1,4,5 | C 07 D 239/60<br>C 07 D 239/34<br>C 07 D 239/553<br>C 07 D 239/46<br>C 07 D 251/30<br>C 07 D 251/38<br>A 01 N 43/54<br>A 01 N 43/66 |
| A | EP - A2 - 0 287 072 (KUMIAI) * Claims 1,13 * -- | 1,4,5 | |
| D,A | EP - A1 - 0 249 707 (KUMIAI) * Claims 1,10,11 * -- | 1,4,5 | |
| D,A | EP - A1 - 0 249 708 (KUMIAI) * Claims 1,13,14 * ---- | 1,4,5 | |

TECHNICAL FIELDS SEARCHED (Int. Cl.5)

C 07 D 239/00
C 07 D 251/00
A 01 N 43/00

The present search report has been drawn up for all claims

| Place of search | Date of completion of the search | Examiner |
|---|---|---|
| WIEN | 12-03-1991 | LUX |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)